# EUROPEAN PATENT APPLICATION

(11) **EP 4 524 122 A1**
(43) Date of publication of application: **19.03.2025**
(21) Application number: 23803757.6
(22) Date of filing: 03.05.2023
(51) Int. Cl.: C07C 29/76, C07C 29/149, C07C 35/14

(54) **METHOD FOR PURIFYING 1, 4-CYCLOHEXANEDIMETHANOL COMPOSITION**

(30) Priority: 10.05.2022 KR 20220057068
(71) Applicant: Hanwha Solutions Corporation, Jung-gu Seoul 04541 (KR)
(72) Inventor: LEE, Sun Uk, Daejeon 34128 (KR); JANG, Namjin, Daejeon 34128 (KR)
(74) Representative: Berggren Oy
(86) International application number: PCT/KR2023/006070
(87) International publication number: WO 2023/219334

(57) **Abstract**

The present disclosure relates to a method for purifying a 1,4-cyclohexanedimethanol (CHDM) composition, and more particularly, to a method for purifying CHDM that can efficiently remove water as a solvent with low energy and can separate high-purity CHDM.

## Description

### [TECHNICAL FIELD]

### CROSS-REFERENCE TO RELATED APPLICATION(S)

This application claims priority to and the benefit of Korean Patent Application No. 10-2022-00570687, filed on May 10, 2022, with the Korean Intellectual Property Office, the disclosure of which is incorporated herein by reference in its entirety.

The present disclosure relates to a method for purifying 1,4-cyclohexanedimethanol(CHDM) composition, and more particularly, to a method for purifying CHDM that can efficiently remove water as a solvent with low energy and can separate high-purity CHDM.

### [BACKGROUND ART]

1,4-Cyclohexanedimethanol(CHDM) is widely used as a raw material for medicines, synthetic resins, synthetic fibers, or dyes, and particularly, is used as a raw material for polyethylene terephthalate, which is an environmentally friendly polyester.

1,4-Cyclohexanedimethanol exists as stereoisomers of cis- and trans-forms, and in order to realize higher quality product, it is required to have higher ratio of trans 1,4-cyclohexanedimethanol (trans CHDM) than cis CHDM.

Among the methods for producing 1,4-cyclohexanedimethanol, there is a method by which phthalate is first hydrogenated and converted into 1,4-cyclohexanedicarboxylic acid(CHDA), and CHDA is then hydrogenated and converted into CHDM. This method uses a heterogeneous catalyst and consists of two step hydrogenation reactions.

This CHDM production process uses water as a solvent, wherein phthalate, i.e., the starting material, has low solubility in water and thus, after the production of CHDM, about 80% or more of water is present in a reaction system. Since water is a solvent with a high specific heat, removing water through a typical separation process is very inefficient in terms of energy. In addition, CHDM has a hydroxyl group, and thus exhibits high hydrophilicity to water. For this reason, when removing the solvent from the crude product of CHDM and recovering CHDM, there is a high risk that the concentration of CHDM accompanied with water will increase. If the concentration of CHDM accompanied with water increases, the loss of CHDM occurs, which significantly reduces the economic feasibility of the process and also has a negative impact on the environment.

Therefore, there is a need to develop a process for effectively removing a solvent from a crude product obtained after the production of CHDM.

### [DETAILED DESCRIPTION OF THE INVENTION]

### [Technical Problem]

In order to solve the above-mentioned problems, it is an object of the present disclosure to provide a method for purifying a 1,4-cyclohexanedimethanol composition, which can efficiently remove water as a solvent from a 1,4-cyclohexanedimethanol crude composition using low energy, and also has high purity due to low content of by-products and water.

### [Technical Solution]

In order to achieve the above object, according to an aspect of the present disclosure, there is provided a method for purifying a 1,4-cyclohexanedimethanol composition comprising:
a step 1 of introducing a CHDM crude composition containing 1,4-cyclohexanedimethanol(CHDM), water and by-products into an FFE (Falling Film Evaporator), evaporating a first stream containing water as a main component in an upper part of the FFE, and separating a second stream containing CHDM as a main component in a lower part of the EFE;
a step 2 of transferring the first stream to an absorber and recovering CHDM at the lower part of the absorber; and
a step 3 of compressing a stream discharged to the upper part of the absorber in the step 2 using an MVR (Mechanical Vapor Compressor) and using it as a heat source for the FFE.

### [ADVANTAGEOUS EFFECTS]

The method for purifying 1,4-cyclohexanedimethanol of the present disclosure is low in energy consumption, has high removing efficiency of water as a solvent, and thus can produce high-quality 1,4-cyclohexanedimethanol at low cost.

In addition, according to the method for purifying a 1,4-cyclohexanedimethanol composition of the present disclosure, the content of by-products and water is very low and thus can provide 1,4-cyclohexanedimethanol with high purity, which may be expected to improve the physical properties when used as a polymer raw material.

### [BRIEF DESCRIPTION OF THE DRAWINGS]

FIG. 1 is a schematic diagram showing a method for purifying a 1,4-cyclohexanedimethanol composition according to an embodiment of the present disclosure.
FIG. 2 is a schematic diagram showing a method for purifying a 1,4-cyclohexanedimethanol composition according to a comparative example.
FIG. 3 is a schematic diagram showing a method for purifying a 1,4-cyclohexanedimethanol composition according to a comparative example.

### [DETAILED DESCRIPTION OF THE EMBODIMENTS]

The technical terms used herein is only to explain exemplary embodiments and is not intended to limit the scope of the present disclosure. The singular forms "a," "an" and "the" are intended to include plural forms, unless the context clearly indicates otherwise. It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

A method for purifying 1,4-cyclohexanedimethanol composition according to the present disclosure comprises: a step 1 of introducing a CHDM crude composition containing 1,4-cyclohexanedimethanol(CHDM), water and by-products into an FFE (Falling Film Evaporator), evaporating a first stream containing water as a main component in an upper part of the FFE, and separating a second stream containing CHDM as a main component in a lower part of the EFE; a step 2 of transferring the first stream to an absorber and recovering CHDM at the lower part of the absorber; and a step 3 of compressing a stream discharged to the upper part of the absorber in the step 2 using an MVR (Mechanical Vapor Compressor) and using it as a heat source for the FFE.

Hereinafter, the method for purifying a 1,4-cyclohexanedimethanol composition according to an embodiment of the present disclosure will be described in detail in each step with reference to the accompanying drawings.

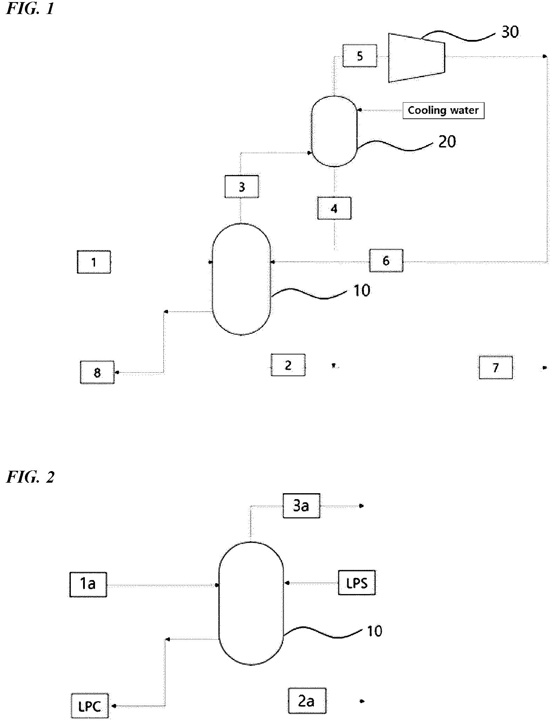

FIG. 1 is a schematic diagram showing a method for purifying a 1,4-cyclohexanedimethanol composition according to an embodiment of the present disclosure.

First, a step 1 of introducing a CHDM crude composition containing 1,4-cyclohexanedimethanol(CHDM), water and by-products into an FFE (Falling Film Evaporator), evaporating a first stream containing water as a main component in an upper part of the FFE, and separating a second stream containing CHDM as a main component in a lower part of the EFE is conducted.

The 1,4-cyclohexanedimethanol crude composition to be purified by the purification method of the present disclosure is obtained by two step hydrogenation reactions of terephthalic acid, but is not limited thereto, wherein the terephthalic acid, which is a first reactant, undergoes a hydrogenation reaction in a state dissolved in water, but has low solubility in water, so that the 1,4-cyclohexanedimethanol crude composition contains a large amount of water.

As an example, the 1,4-cyclohexanedimethanol crude composition contains 10 to 40 wt.% of CHDM, 60 to 90 wt.% of water, and small amounts of by-products based on the total weight. A significant portion of the remaining components except the 10 to 40 wt.% of CHDM is occupied by water.

By the way, since water is a solvent with a high specific heat, removing water through a typical separation process is very inefficient in terms of energy. In addition, CHDM has a hydroxyl group, and thus exhibits high hydrophilicity to water. For this reason, when removing the solvent from the crude product of CHDM and recovering CHDM, there is a high risk that the concentration of CHDM accompanied with water will increase. If the concentration of CHDM accompanied with water increases, the loss of CHDM occurs, which significantly reduces the economic feasibility of the process and also has a negative impact on the environment. Therefore, in order to recover purified CHDM, it is necessary to remove water with low energy and reduce the concentration of CHDM accompanied with water as much as possible.

Thus, in an embodiment of the present disclosure, first, a step of removing water from a CHDM crude composition using a FFE (Falling Film Evaporator) is conducted.

Falling Film Evaporator (FFE) is a type of evaporator, which is a device that evaporates the target material by making it flow down into a thin film in the upper part of a heating tube. The FFE has the advantage that it can realize a continuous evaporation process and evaporates the solvent through a film with a large surface area and a small thickness, thereby allowing for more efficient removal of the solvent than a conventional evaporator.

Referring to FIG. 1, a step 1 of introducing 1,4-cyclohexanedimethanol crude composition 1, which is the purifying target of the present disclosure, into an FFE 10, evaporating a first stream 3 containing water as a main component in an upper part of the FFE 10, and separating a second stream 2 containing CHDM as a main component in a lower part of the EFE is conducted.

In the present disclosure, "containing as a major component" means that the component is contained in an amount of 60 wt.% or more based on the total weight of the stream.

In the present disclosure, "barg" means bar gauge pressure, which is a pressure derived by subtracting an atmospheric pressure from an actual pressure at the measurement location.

As an example, the operation of FFE 10 in the step 1 can be conducted at a temperature of 100 to 130°C under a pressure of 0.1 to 4 barg. More preferably, the operation can be conducted at a temperature of 115 to 125°C under a pressure of 0.5 to 3 barg, or at a temperature of 118 to 123°C under a pressure of 0.5 to 2 barg. In the case of deviating the above-mentioned temperature and pressure conditions, water is not sufficiently removed in the upper part of FFE 10, and thus further removal of water is required in the latter stage process, which may increase the energy consumption required for purification.

By the step 1 as described above, 90 wt.% or more, preferably 92 wt.% or more, more preferably 95 wt.% or more water based on the total weight of the water contained in the initial CHDM crude composition 1 can be evaporated in the upper part of the FFE 10 and separated into the first stream 3. It is preferable that the upper limit of water contained in the first stream 3 is higher, but may be about 99 wt.% or less, or about 98 wt.% or less, in terms of energy efficiency.

In the lower part of the FFE 10, most of the water is removed from the 1,4-cyclohexanedimethanol crude composition 1 before being introduced into the FFE 10, and a second stream 2 containing CHDM as a main component is separated. According to an embodiment of the present disclosure, the second stream 2 may contain 95 wt.% or more, preferably 96 wt.% or more, and more preferably 98 wt.% or more of CHDM based on the total weight of CHDM contained in the initial CHDM crude composition 1. It is preferable that the upper limit of CHDM contained in the second stream 2 is higher, but may be about 99.9 wt.% or less, or about 99.5 wt.% or less in terms of energy efficiency.

Next, a step 2 of transferring the first stream 3 separated in the upper part of the FFE 10 to an absorber 20 and recovering CHDM at the lower part of the absorber is conducted.

In the step 1, as most of the water is separated into the first stream 3 from the CHDM crude composition using the FFE 10, most of the water contained in the initial CHDM crude composition 1 is separated into the first stream 3, and a second stream 2 of purified CHDM may be separated at the lower part, but the first stream 3 separated at the upper part still contains about 1 wt.% of CHDM based on the total weight of CHDM contained in the initial CHDM crude composition 1.

In the step 2, this excess CHDM contained in the first stream 3 is recovered at the absorber 20.

That is, the step 2 is a step in which the first stream 3 that has undergone the separation of the step 1 using FFE 10 is distilled in the absorber 20 to separate by-products and water at the upper portion, and separate and recover CHDM at the lower part.

At this time, cooling water at 20 to 50°C, preferably 25 to 35°C, may can be introduced into the absorber 20 of the step 2, and the amount of CHDM recovered may increase depending on the amount of cooling water introduced. Meanwhile, the CHDM stream 4 recovered is merged with the second stream 2 separated at the lower part of the FFE 10 in the step 1 and included in the CHDM concentrated stream 7, so that the more cooling water is used, the more energy is required for processing in the latter stage process, which reduces economic efficiency, and thus it is necessary to use an appropriate amount.

Thus, according to an embodiment of the present disclosure, when the amount of CHDM contained in the first stream 3 introduced into the absorber 20 is about 15 to 25 kg/h, cooling water is introduced in an amount of about 100 to 200 kg/hr, preferably about 120 to 180 kg/hr, into the absorber 20, which may be preferable in terms of both CHDM recovery rate and economic efficiency.

According to an embodiment of the present disclosure, in the step 2, 50 wt.% or more, preferably 55 wt.% or more, more preferably 60 wt.% or more of CHDM based on the total weight of CHDM contained in the first stream 3 introduced into the absorber 20 can be recovered into a CHDM stream 4. It is preferable that the upper limit of CHDM recovered as the CHDM stream 4 is higher, but may be about 90 wt.% or less, or about 80 wt.% or less in terms of energy efficiency.

The recovered CHDM stream 4 is merged with the second stream 2 separated at the lower part of the FFE 10 in the step 1 and sent to the CHDM concentrated stream 7, and then undergoes the latter stage process such as distillation to obtain CHDM with a purity of 99.8% or more.

Next, a step 3 of compressing the third stream 5 discharged to the upper part of the absorber 20 in the step 2 using an MVR (Mechanical Vapor Compressor) and using it as a heat source 6 for the FFE is conducted.

The third stream 5 discharged to the upper part of the absorber 20 is mostly composed of water in a gaseous state (about 99 wt.% or more) and contains a small amount of CHDM and by-products.

If this is compressed using the MVR 30 and then circulated to the FFE 10 and used as a heat source 6 for the FFE 10, the amount of steam consumed in the FFE 10 can be reduced, and thus the amount of energy used can be greatly reduced.

The CHDM composition obtained by the purification method of the present disclosure as described above may have a CHDM purity of 99.5 wt.% or more, calculated from the CHDM content measured by a gas chromatography (GC) analysis.

More specifically, in the purified CHDM composition, the content of CHDM, i.e., the purity, may be 99.5 wt.% or more, or 99.7 wt.% or more, or 99.75 wt.% or more, or 99.8 wt.% or more.

In addition, the content of water remaining in the purified CHDM composition is 0.15 wt.% or less, or 0.13 wt.% or less, or 0.12 wt.% or less, or 0.10 wt.% or less, or 0.09 wt.% or less, or 0.08 wt.% or less, or 0.07 wt.% or less, based on the total weight of the purified CHDM composition, and the water content may be very low.

As described above, CHDM obtained by the purification method of the present disclosure has high purity and contains very low amounts of residual water and by-products, and thus can be usefully used as a raw material for producing high-quality products such as medicines, synthetic resins, synthetic fibers, or dyes.

The crude 1,4-cyclohexanedimethanol crude composition, which is the target of the purification method of the present disclosure described above, can be produced by performing a first-step hydrogenation reaction of terephthalic acid as the starting material to 1,4-cyclohexanedicarboxylic acid (CHDA), and a second-step hydrogenation reaction from 1,4-cyclohexanedicarboxylic acid to 1,4-cyclohexanedimethanol.

As an example, it may be produced by a production method comprising: a step 1 of introducing a reaction solution containing terephthalic acid, a first hydrogenation catalyst, and water, and hydrogen gas into a first reactor equipped with a stirrer, and performing a hydrogenation reaction to produce 1,4-cyclohexane dicarboxylic acid (CHDA) containing cis isomers and trans isomers; and a step 2 of introducing a reaction product of the step 1, a reaction solution containing a second hydrogenation catalyst, and water, and hydrogen gas into a second reactor equipped with a stirrer, and performing a hydrogenation reaction to produce 1,4-cyclohexanedimethanol(CHDM) containing cis isomers and trans isomers, but the present disclosure is not limited thereto.

The crude composition of 1,4-cyclohexanedimethanol, which is a reaction product obtained after the step 2 reaction, includes 1,4-cyclohexanedimethanol containing cis isomers and trans isomers, water as a solvent, and reaction by-products, etc., and this may be purified using the purification method of the present disclosure to obtain high-purity 1,4-cyclohexanedimethanol.

Hereinafter, the present disclosure will be explained in more detail through examples for better understanding of the invention. However, these examples are presented for illustrative purposes only, and the present disclosure is not limited thereby.

### <EXAMPLE>

### Production Example 1

### Step 1

A first reactor equipped with a gas-induced type stirrer was prepared.

In the reactor, 3,200 g of terephthalic acid(TPA, 5 wt.%), 16,000 kg of distilled water as a solvent, and 15 mass% of hydrogenation catalyst Pd/C (containing 5 wt% of Pd, based on a carrier carbon) based on the total solution containing the reactants and solvent were introduced as reactants, and the internal atmosphere of the reactor was replaced with nitrogen, and then, the temperature of the mixed solution was raised to 170°C while stirring at low speed.

After the temperature of the mixed solution reached 170°C, in order to dissolve TPA, the solution was stirred for 30 minutes while maintaining the temperature. And then, the stirring speed was increased, and while supplying hydrogen gas in the reaction solution such that the internal pressure of the reactor was maintained at 100 barg, and surface area per unit volume of hydrogen gas was maintained at 300 to 500 m²/m³, a hydrogen reaction was conducted for 3 hours.

After the reaction was completed, a product comprising 3,144 g of 1,4-cyclohexanedimethanol(CHDA) (trans CHDA rate in CHDA: 68 wt.%) and 16,031g of water was obtained, and it was used as the reactant of the hydrogenation reaction of step 2, after removing only the hydrogenation catalyst by a metal filter.

### Step 2

A second reactor equipped with a gas-induced type stirrer was prepared.

In the second reactor, 3,144 g of the reaction product of the step 1, i.e., CHDA (trans CHDA rate in CHDA: 68 wt. %), 16,031 g of distilled water solvent, and about 57 mass% of a catalyst(ruthenium-tin/carbon catalyst, containing 5 parts by weight of ruthenium, and 5.8 parts by weight of tin, based on 100 parts by weight of a carbon carrier) based on the total solution containing the reactants and solvent were introduced, purged twice with nitrogen of 5 bar, and purged twice with hydrogen of 5 bar, and then, the temperature was raised to 230°C while stirring at low speed under hydrogen atmosphere (about 14~15 bar).

After reaching the reaction temperature, hydrogen was introduced to the reaction pressure of 100 barg, and then, the stirring speed was increased, and a reaction was conducted for 6 hours while maintaining the surface area per unit volume of hydrogen gas bubbles at 300 to 450 m²/m³.

The hydrogenation catalyst was removed in the reaction product of the step 2 by a metal filter, and then the product was transferred to the purification step of Example.

### Comparative Example 1

In accordance with the schematic diagram shown in FIG. 2, the 1,4-cyclohexanedimethanol composition was purified.

First, the CHDM crude composition 1a of Production Example 1 was introduced into a FFE 10. Water was removed in the upper stream 3a of the FFE 10, and CHDM was separated in the lower stream 2a. When the FFE 10 was operated so that about 10% of water was present in the lower stream 2a, the required heat capacity was about 5 Gcal/hr, and LPS (low pressure steam) was used as a heat source. At this time, about 20 kg/hr of CHDM was accompanied in the upper stream 3a of the FFE 10. The upper stream 3a was then treated as wastewater, and the organic matter content in the upper stream 3a was 5717 ppm.

The temperature, pressure, and composition in each stream are shown in Table 1 below.

**[Table 1]**

| Stream | 1a | 2a | 3a |
|---|---|---|---|
| Temperature (°C) | 121 | 120.4 | 103 |
| Pressure (barg) | 1 | 1 | 0.1 |
| Water (kg/hr) | 10088.7 | 350.4 | 9738.3 |
| CHDM(kg/hr) | 2556.0 | 2535.8 | 20.2 |
| By-product (kg/hr) | 140.0 | 104.2 | 35.8 |

### Comparative Example 2

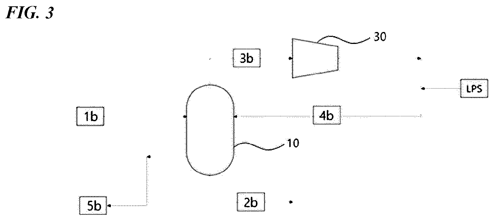

In accordance with the schematic diagram shown in FIG. 3, the 1,4-cyclohexanedimethanol composition was purified.

First, the CHDM crude composition 1b of Production Example 1 was introduced into a FFE 10. Water was removed in the upper stream 3b of the FFE 10, and CHDM was separated in the lower stream 2b. When the FFE 10 was operated so that about 10% of water was present in the lower stream 2b, the required heat capacity was about 5 Gcal/hr, and LPS (low pressure steam) was used as an initial heat source. At this time, about 20 kg/hr of CHDM was accompanied even in the upper stream 3b .

The upper stream 3b was sent to the MVR 30 and used again as the heat source 4b of the FFE 10 through gas phase compression. The heat capacity capable of being obtained using the MVR 30 was about 5.3 Gcal/hr, and operation was possible without inputting additional heat capacity. The electricity required at this time was about 730 kW, which was equivalent to 0.63 Gcal/hr, and thus even taking into account the electricity usage, the introduction of MVR30 allowed for a significant reduction in energy usage compared to Comparative Example 1. However, there was still a considerable amount of CHDM (35.8 kg/hr) that was not recovered due to being accompanied in the upper stream 3b, so there was no advantage in terms of the recovery rate of CHDM.

The temperature, pressure, and composition in each stream are shown in Table 2 below.

**[Table 2]**

| Stream | 1b | 2b | 3b | 4b | 5b |
|---|---|---|---|---|---|
| Temperature (°C) | 121 | 120.4 | 103 | 156 | 110.5 |
| Pressure (barg) | 1 | 1 | 0.1 | 2.4 | 2.4 |
| Water (kg/hr) | 10088.7 | 350.4 | 9738.3 | 9738.3 | 9738.3 |
| CHDM (kg/hr) | 2556.0 | 2535.8 | 20.2 | 20.2 | 20.2 |
| By-product (kg/hr) | 140.0 | 104.2 | 35.8 | 35.8 | 35.8 |

### Example 1

In accordance with the schematic diagram shown in FIG. 1, the 1,4-cyclohexanedimethanol composition was purified.

The CHDM crude composition 1 of Production Example 1 was introduced into a FFE 10. Water was removed in a first stream 3 at the upper part of the FFE 10, and CHDM was separated in a second stream 2 at the lower part. When the FFE 10 was operated so that about 10% of water was present in the second stream 2, the required heat capacity was about 5 Gcal/hr, and LPS (low pressure steam) was used as an initial heat source. At this time, about 20 kg/hr of CHDM was accompanied even in the first stream 3.

Next, the first stream 3 separated in the upper part of the FFE 10 was transferred to the absorber 20. Cooling water at 30°C was introduced at about 150 kg/hr into the absorber 20.

The third stream 5 discharged to the upper part of the absorber 20 was compressed by the MVR 30 and used again as the heat source 6 of the FFE, and was recovered into the CHDM stream 4 at the lower part.

Although the upper stream 3b of the previous Comparative Example 2 contained about 20 kg/hr of CHDM, it was confirmed that in the case of the third stream 5 fed to the MVR 30, about 12 kg/hr of CHDM, including about 8 kg/hr of CHDM, was re-recovered in the lower CHDM stream 4.

The heat capacity capable of being obtained using the MVR 30 was about 5.3 Gcal/hr, and operation was possible without inputting additional heat capacity. The electricity required at this time was about 730 kW, which was equivalent to 0.63 Gcal/hr, and thus even taking into account the electricity usage, the introduction of MVR30 allowed for a significant reduction in energy usage compared to Comparative Example 1.

In addition, the organic matter content in the stream being treated was 4454 ppm, which was lower than the organic matter content of 5717 ppm in Comparative Example 1.

The temperature, pressure, and composition in each stream are shown in Table 3 below.

**[Table 3]**

| | | | | | | | | |
|---|---|---|---|---|---|---|---|---|
| Stream | 1 | 2 | 3 | 4 | 5 | 6 | 7 | 8 |

| | | | | | | | | |
|---|---|---|---|---|---|---|---|---|
| Temperature (°C) | 121 | 120.4 | 103 | 103 | 103 | 156 | 101 | 110.5 |
| Pressure (barg) | 1 | 1 | 0.1 | 0.1 | 0.1 | 2.4 | 0.1 | 2.4 |
| Water (kg/hr) | 10088.7 | 350.4 | 9738.3 | 166.4 | 9721.9 | 9721.9 | 516.8 | 9721.9 |
| CHDM (kg/hr) | 2556.0 | 2535.8 | 20.2 | 12.3 | 7.9 | 7.9 | 2548.1 | 7.9 |
| By-product (kg/hr) | 140.0 | 104.2 | 35.8 | 0.2 | 35.6 | 35.6 | 104.4 | 35.6 |

According to the purification method according to Examples of the present disclosure as described above, it was confirmed that by using the FFE, the absorption tower, and the MVR, water can be efficiently removed with low energy, high purity CHDM can be separated, and the organic matter content in wastewater can be reduced.

### [Description of Reference Numerals]

1: 1,4-cyclohexanedimethanol crude composition
2: second stream
3: first stream
4: CHDM stream
5: third stream
6: heat source
7: CHDM concentrated stream
10: FFE (Falling Film Evaporator)
20: absorber
30: MVR (Mechanical Vapor Compressor)

## Claims

1. A method for purifying a 1,4-cyclohexanedimethanol composition comprising:
a step 1 of introducing a CHDM crude composition containing 1,4-cyclohexanedimethanol(CHDM), water and by-products into an FFE (Falling Film Evaporator), evaporating a first stream containing water as a main component in an upper part of the FFE, and separating a second stream containing CHDM as a main component in a lower part of the EFE;
a step 2 of transferring the first stream to an absorber and recovering CHDM at the lower part of the absorber; and
a step 3 of compressing a stream discharged to the upper part of the absorber in the step 2 using an MVR (Mechanical Vapor Compressor) and using it as a heat source for the FFE.

2. The method for purifying a 1,4-cyclohexanedimethanol composition according to claim 1, wherein operation of the FFE is conducted at a temperature of 100 to 130°C under a pressure of 0.1 to 4 barg.

3. The method for purifying a 1,4-cyclohexanedimethanol composition according to claim 1, wherein the CHDM crude composition comprises 10 to 40 wt.% of CHDM, 60 to 90 wt.% of water and by-products based on the total weight.

4. The method for purifying a 1,4-cyclohexanedimethanol composition according to claim 1, wherein by the step 1, 90 wt.% or more of water is separated into the first stream, based on the total weight of water contained in the initial CHDM crude composition.

5. The method for purifying a 1,4-cyclohexanedimethanol composition according to claim 1, wherein by the step 1, 95 wt.% or more of CHDM is separated into the second stream, based on the total weight of CHDM contained in the initial CHDM crude composition.

6. The method for purifying a 1,4-cyclohexanedimethanol composition according to claim 1, wherein cooling water at 20 to 50°C is introduced into the absorber.

7. The method for purifying a 1,4-cyclohexanedimethanol composition according to claim 6, wherein, when the amount of CHDM contained in the first stream introduced into the absorber is 15 to 25 kg/hr, 100 to 200 kg/hr of cooling water is introduced into the absorber.

8. The method for purifying a 1,4-cyclohexanedimethanol composition according to claim 1, wherein by the step 2, 50 wt.% or more of CHDM is recovered into a CHDM stream, based on the total weight of CHDM contained in the first stream introduced into the absorber.

9. The method for purifying a 1,4-cyclohexanedimethanol composition according to claim 8, wherein the recovered CHDM stream is merged with the second stream separated from the lower part of the FFE in the step 1 and sent to a CHDM concentrated stream.

10. The method for purifying a 1,4-cyclohexanedimethanol composition according to claim 1, wherein the CHDM crude composition is produced by performing:
a first-step hydrogenation reaction of terephthalic acid as a starting material into 1,4-cyclohexane dicarboxylic acid (CHDA), and a second-step hydrogenation reaction of 1,4-cyclohexane dicarboxylic acid into 1,4-cyclohexanedimethanol.
